# EUROPEAN PATENT SPECIFICATION

(11) **EP 4 581 639 B1**
(45) Date of publication and mention of the grant of the patent: **07.10.2026**
(21) Application number: 23762479.6
(22) Date of filing: 30.08.2023
(51) Int. Cl.: G16H 20/40, G16H 40/20, A61B 34/20, G01T 1/00

(54) **GUIDANCE IN SPATIAL SETUPS OF A ROOM IN A MEDICAL FACILITY**
FÜHRUNG FÜR RÄUMLICHE EINRICHTUNG EINES RAUMS IN EINER MEDIZINISCHEN EINRICHTUNG
GUIDAGE DANS DES CONFIGURATIONS SPATIALES D'UNE PIÈCE DANS UNE INSTALLATION MÉDICALE

(30) Priority: 02.09.2022 US 202263403354 P; 06.01.2023 EP 23150528
(43) Date of publication of application: 09.07.2025
(73) Proprietor: Koninklijke Philips N.V., 5656 AE Eindhoven (NL)
(72) Inventor: BUIL, Vincentius Paulus, 5656 AG Eindhoven (NL); WEBB, Penelope Eugenia, 5656 AG Eindhoven (NL); PRINTZ, Adam, 5656 AG Eindhoven (NL); KUHLMANN, Daan, 5656 AG Eindhoven (NL); WIJN, Victor, 5656 AG Eindhoven (NL)
(74) Representative: Philips Intellectual Property & Standards
(86) International application number: PCT/EP2023/073773
(87) International publication number: WO 2024/047098

(56) References cited:
- US-A1- 2022 223 268

## Description

### FIELD OF THE INVENTION

The present invention relates to a device for guidance in spatial setups of a room in a medical facility, to a system for guidance in spatial setups of a room in a medical facility and to a method for guidance in spatial setups of a room in a medical facility.

### BACKGROUND OF THE INVENTION

In minimally invasive and other types of surgery, a plurality of staff, i.e. people, and a number of pieces of equipment are positioned in various configurations, dependent on the type and variant of a procedure and the phase of the particular procedure. For example, in cardiac procedures such as trans-aortic valve implantation (TAVI), the patient bed, C-arm X-ray device, X-ray protection glasses, anaesthesia equipment, contrast pump, perfusion device, ultrasound device, display monitors etc., together with the interventional cardiologist, cardiac surgeon, surgical assistant, fellow, aneasthesiologist, echographer, perfusionist, preparation nurse and circular nurse, are changing positions dependent on the phase the TAVI procedure is in, and dependent whether it is a trans apical or trans femoral procedure, for example. In particular, orientations of the equipment and people may change during the procedures, e.g. a C-arm imaging system is positioned differently dependent on the type of procedure. Further, many more types of procedures exist, e.g. cardiac, peripheral, neuro, and many more, during which people and equipment need to reposition between procedures and between phases within those procedures. It has been shown that this is time consuming and may result in inefficiencies, errors, and impractical setups, especially when inexperienced staff is involved.

US 2022/223268 A1, according to its abstract, describes a method for optimizing a medical procedure room set up that comprises obtaining a medical procedure room setup input associated with a medical practitioner identifier, a medical procedure identifier, and a medical procedure room identifier for the medical procedure room set up. A virtual image guidance for the medical procedure room set up based on the received input is provided. The method comprises acquiring data associated with one or more of item, equipment, and personnel in the medical procedure room setup and analyzing prestored data associated with item, equipment, and personnel in one or more reference medical procedure room setups corresponding to the received medical procedure room setup input. The method comprises comparing the acquired data with the analyzed prestored data associated with the respective item, equipment, and personnel in the one or more reference medical procedure room setups and providing recommendation for optimizing the medical procedure room set up.

### SUMMARY OF THE INVENTION

There may thus be a need to provide improvements for a facilitated work procedure in medical interventions.

The object of the present invention is solved by the subject-matter of the independent claims; further embodiments are incorporated in the dependent claims. It should be noted that the following described aspects of the invention apply also for the device for guidance in spatial setups of a room in a medical facility, for the system for guidance in spatial setups of a room in a medical facility and for the method for guidance in spatial setups of a room in a medical facility.

According to the present invention, a device according to claim 1 for guidance in spatial setups of a room in a medical facility is provided. The device comprises a data input, a data processor and an output interface. The data input is configured to provide procedure data about a current executed medical procedure. The data input is also configured to provide spatial data of at least one of the group of staff and equipment relating to the currently executed medical procedure. The data processor is configured to calculate upcoming physical movement of at least one of the group of the staff and the equipment based on the procedure data and the spatial data procedure data. The data processor is also configured to determine, based on the calculated upcoming physical movement, at least one of the group of equipment movement instructions and staff spatial guidance. The output interface is configured to provide at least one of the group of the equipment movement instructions and the staff spatial guidance.

As an effect, the working scenario in the room in a medical facility can be organized and arranged in a way that matches best with the respective procedure performed. The prediction of upcoming movement allows to determine the optimized movement pattern, since the device is capable of having an overview, whereas the individual staff member is focused on a subjective view, from which he has to consider what movement will take place in the room. By providing equipment movement instructions and staff spatial guidance, staff members are relieved of that burden and can concentrate more on their respective tasks. The equipment movement instructions ensure an optimized arrangement of movable devices and minimum disturbance in their interaction with other movable parts and staff members. Equipment can thus be used in the best and most efficient way. This facilitates the workflow in general and provides benefits in terms of staff member comfort, safety and efficiency.

According to the invention, for the provision of the procedure data about the current executed medical procedure, the data processor is configured to track type and phase of the current executed medical procedure.

According to the invention, for the tracking of the type and phase of the current executed medical procedure, the data processor is configured to provide at least one of the group of: procedure analysis based on image data of a current scenario, procedure analysis based on acoustic data comprising at least parts of a conversation by the staff of a current scenario, and patient related data.

According to an example, for the calculation of the upcoming physical movement, the data processor is configured to determine possible upcoming target positions of at least one of the group of the staff and the equipment based on the procedure data and the spatial data procedure data. The data processor is also configured to calculate possible movement trajectories. The data processor is also configured to provide a plausibility check to select a target position and a trajectory. According to an option, provided in addition or alternatively, the data processor is configured to calculate the upcoming physical movement comprising at least one of the group of from-to routes for at least one of the group of the staff and the equipment and an order for the movements of at least one of the group of the staff and the equipment.

According to an example, as further input, the data input is configured to provide radiation dose information for at least a part of the staff members, and wherein, based on the respective location, the data processor is configured to compute a dose exposure probability and to determine an adjusted position information to reduce radiation exposure of the staff.

According to an example, for determining the equipment instructions and the staff spatial guidance, the data processor is configured to provide a parameter weighting to achieve an overall optimal score.

According to an example, in a first option, the data processor is configured to detect, based on changes and/or input by the user, deviations from at least one of the group of the provided equipment movement instructions and the staff spatial guidance. The data processor is also configured to propose changes to stored room settings to the user providing a learning update. In a second option, provided in addition or as an alternative, the data processor is configured to detect, based on tracked patient vital parameters, changes above a predetermined threshold indicating a possible upcoming emergency scenario. The data processor is also configured to provide emergency equipment movement instructions and emergency staff spatial guidance.

According to an example, the data-processor is configured: to calculate, based on the provided procedure data, the spatial data and the calculated upcoming physical movement, alternative positions under the doctrine that at least a part of the group of staff and equipment is organized according to a flock-of-birds concept; to assess if any of the alternative positions provides benefits for upcoming stages; and to provide at least one of the group of adjusted equipment movement instructions and adjusted staff spatial guidance.

In an example, several devices are provided. At least a part of the movable participants comprising staff and equipment is assigned with an individual device such that these can optimize their position under the flock-of-birds concept, also known as flocking. The (moving) participants achieve optimized positions regarding the actual moving forward in the context of a plurality of neighboring moving participants.

In another example, specific equipment is equipped with its own associated individual behavioral intelligence (IBI) to physically follow a certain staff member. This IBI uses input from the central system on locations of other equipment and staff to drive to locations near that staff member while avoiding collisions with other equipment and staff. The specific equipment may also have its own sensors to avoid collisions with other equipment and staff. Optionally, the central system may anticipate what the specific equipment will do, knowing its rule set, and as such assist in avoiding collisions during the movement planning and execution for the other equipment and staff.

According to the present invention, also a system for guidance in spatial setups of a room in a medical facility is provided. The system comprises a device according to one of the preceding examples and option. The system further comprises a capturing arrangement. The capturing arrangement is configured to capture a current executed medical procedure and to detect spatial location and arrangement of at least one of the group of staff and equipment. The device for guidance is configured to determine the procedure data based on the captured current executed medical procedure. The device for guidance is also configured to determine the spatial data of the staff and/or equipment based on the detected spatial location and arrangement of at least one of the group of staff and equipment.

According to an example, the system further comprises equipment. At least a part of the equipment is provided with at least one of the group of: i) a drivable base, wherein the equipment movement instructions are provided to operate the base to move the equipment accordingly; ii) an actuatable boom, wherein the equipment movement instructions are provided to actuate the boom to reposition the equipment accordingly; and iii) an actuatable pan-tilt mechanism, wherein the equipment movement instructions are provided to actuate the pan-tilt mechanism to adjust the equipment accordingly. At least a part of the equipment is configured to be moved automatically according to the movement instructions.

In an example, at least part of the staff and the equipment, as participants in a procedure, is organized under a flock-of-birds concept. According to this concept, at least two of the participants, comprising at least two members of a group comprising at least one of the staff member and the equipment, are provided with its own algorithm running on a processor to optimize its position in the flock.

According to the present invention, also a method according to claim 11 for guidance in spatial setups of a room in a medical facility is provided. The method comprises the following steps:
- providing procedure data about a current executed medical procedure;
- providing spatial data of at least one of the group of staff and equipment relating to the currently executed medical procedure;
- calculating upcoming physical movement of at least one of the group of the staff and the equipment based on the procedure data and the spatial data procedure data;
- determining, based on the calculated upcoming physical movement, at least one of the group of equipment movement instructions and staff spatial guidance; and
- providing at least one of the group of the equipment movement instructions and the staff spatial guidance.

A field of application is rooms like operation rooms in hospitals or private clinics, for all types of surgery, including cath-labs and hybrid labs, or in emergency rooms, intensive care units, general wards, or any other care setting where multiple staff members and pieces of equipment come together to execute medical procedures.

According to an aspect, the procedure type and phase are tracked during operation room (OR) activities. As an option, room layout configurations per procedure type and phase are provided. Further, staff and equipment positions and orientations are tracked. From-to routes for staff and equipment for phase transitions requiring different staff and equipment positions and orientations are calculated. Staff guidance and feedback is provided, for example, via a user interface on equipment movement and when and where the staff should reposition. The equipment on motorized wheels is moved, and motorized booms or pan-tilt mechanisms are brought to the desired positions and orientations.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for guidance in spatial setups of a room in a medical facility like an operation room.
Fig. 2 shows an example of a system for guidance in spatial setups of a room in a medical facility.
Fig. 3 shows another example of the system for guidance where a part of the equipment is provided with a motorized base.
Fig. 4 shows a further example of the system for guidance with indicated projections or target positions for a part of the equipment.
Fig. 5 shows four different scenarios during a procedure in a room in a medical facility.
Fig. 6 shows basic steps of an example of a method for guidance in spatial setups of a room in a medical facility.
Fig. 7 shows a schematic setup and workflow diagram of an example for guidance in spatial setups of a room in a medical facility.
Fig. 8 shows an option for the setup and workflow of Fig. 7.
Fig. 9 shows a further option for the setup and workflow of Fig. 7.
Fig. 10 shows another option for the setup and workflow of Fig. 7.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

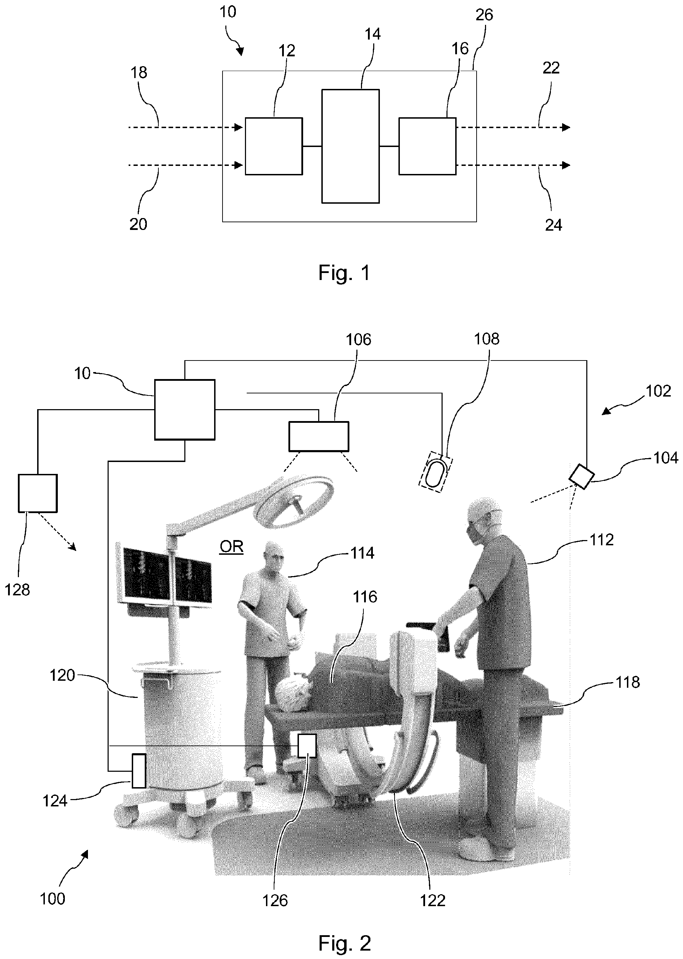

Fig. 1 schematically shows an example of a device 10 for guidance in spatial setups of a room in a medical facility. The device 10 comprises a data input 12, a data processor 14 and an output interface 16. The data input 12 is configured to provide procedure data 18 about a current executed medical procedure. The data input 12 is also configured to provide spatial data 20 of at least one of the group of staff and equipment relating to the currently executed medical procedure. The data processor 14 is configured to calculate upcoming physical movement of at least one of the group of the staff and the equipment based on the procedure data 18 and the spatial data procedure data 20. The data processor 14 is also configured to determine, based on the calculated upcoming physical movement, at least one of the group of equipment movement instructions 22 and staff spatial guidance 24. The output interface 16 is configured to provide at least one of the group of the equipment movement instructions 22 and the staff spatial guidance 24.

In the example shown, the data input 12, the data processor 14 and the output interface 16 are provided in an integrated manner in a common structure, such as a housing, as indicated by frame 26. In another example, not shown, the data input 12, the data processor 14 and the output interface 16 are provided separately.

The term "data input" relates to providing or supplying data for data processing steps. The data input 12 can also be referred to as procedure data input. The data input 12 can also be referred to as data supply, as spatial data supply, as input unit or simply as input. In an example, the data input 12 is data-connectable to a data network arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to a user interface arrangement or user interaction device. In another example, the output interface 16 is data-connected to a display. As an example, the signals by the processor can be provided by the output interface 16.

The term "procedure data" relates to all information that allows to retrieve, i.e. to determine the currently performed procedure. The procedure data 18 may comprise information about a current personnel scenario, medical equipment that allows identification about the performed tasks, subject or staff data, and any other features.

The term "scenario" relates to a stage during an operation procedure. The scenario refers to the different setups that are needed during the intervention.

The term "spatial data" relates to information about an arrangement of equipment or staff within the room.

The term "upcoming physical movement" relates to what will happen within the current scenario as a next action regarding the movement of the devices or persons.

The term "equipment movement instruction" relates to moving, adapting, adjusting, changing or actuating movable, adaptable, adjustable, changeable or actuatable equipment.

The term "staff spatial guidance" relates to information for selected or all staff members to achieve a position within the room that has been defined as a temporarily target position.

The term "room in a medical facility" relates to spaces, i.e. rooms, used for medical interventions, medical examinations, medical treatments and the like. Examples for such rooms are operation rooms, i.e. rooms configured for performing medical operations like surgery, and cathlabs, i.e. laboratories configured for performing interventions involving catheters. However, "room in a medical facility" also relates to other rooms that are used for similar purposes and in which a number of staff members and a plurality of movable devices are present during a medical procedure and change their positions during the procedure, such as for example emergency rooms and intensive care units.

As a sort of start, an understanding of a currently performed procedure and a current scenario or situation in an operation room or other room in a in a medical facility is provided. This understanding is taken to determine possible further scenarios or situations in view of the conducted, i.e. performed medical procedure, like an operation. In other words, the further procedure is predicted. Based upon this prediction, changes in location and arrangement of staff and/or equipment are computed. Respective moving instructions or change instructions are generated and provided to be used for moving or changing the spatial situation according to the next scenario of the medical procedure.

The understanding may be provided by respective data. In an option, the understanding is generated by detecting the current situation, e.g. by optical cameras. Further, also other data sources like the conversation among the staff members or stored information about the subject and the planned procedure are used for achieving the understanding of the current situation in the context of the respective procedure.

In an example, the medical procedure takes place in an operational room.

In an example, spatial data relating to the currently executed medical procedure is provided for staff and equipment.

In an example, equipment movement instructions and staff spatial guidance are determined.

In an example, in addition to position determination of staff and equipment, movement patterns are tracked to determine a current executed medical procedure, to provide the procedure data.

In an example, for the provision of the procedure data about the current executed medical procedure, the data processor 14 is configured to track type and phase of the current executed medical procedure.

The term "type" relates to the kind or category of the medical procedure. The term "phase" relates to a certain identifiable part, portion or sequence within the medical procedure.

In an example, for the tracking of the type and phase of the current executed medical procedure, the data processor 14 is configured to provide at least one of the group of procedure analysis based on image data of a current scenario, procedure analysis based on acoustic data comprising at least parts of a conversation by the staff of a current scenario and patient related data.

In an example, the patient related data comprises the type of operation currently performed. In another example, the patient related data comprises current, i.e. updated data, that allows retrieving the phase of the operation currently performed. As an example for updated data, medical data of the patient is monitored, or equipment activity is monitored such that a documentation of certain operational steps allows retrieving the phase of the operation currently performed.

In an example, this also includes input on the procedure data in the form of knowledge. As an example, an input is provided about what procedure is done at the moment, e.g. from electronic medical record (EMR), and what such procedure constitutes of, e.g. which steps. In an option, this can also be stored in e.g. a database. In an option, the image and acoustic data is then used to recognize transitions in steps of the procedure.

In an example, for the provision of the spatial data, the data processor 14 is configured to provide at least one of the group of optical tracking of physical objects in a predetermined space of the room, like an operation room, based on optical image data and marker tracking of at least one of the group of the staff and the equipment. The markers are provided as at least one of the group of optical markers, electromagnetic markers and acoustic markers.

In an example, the staff and the equipment are tracked. The markers can be referred to as beacons, e.g. attached to staff or equipment.

In an example, for the calculation of the upcoming physical movement, the data processor 14 is configured to determine possible upcoming target positions of at least one of the group of the staff and the equipment based on the procedure data and the spatial data procedure data. The data processor 14 is also configured to calculate possible movement trajectories. The data processor 14 is furthermore configured to provide a plausibility check to select a target position and a trajectory.

In an example, for the calculating of the upcoming physical movement of staff and/or equipment, an anticipation of an expected next scenario of the executed medical procedure is computed.

In an example, the information of where people and systems are preferably located per step in a procedure, is predefined and available from a database.

In another example, this information is learned over time and then stored.

In an example, the data processor 14 is configured to calculate the upcoming physical movement comprising at least one of the group of from-to routes for at least one of the group of the staff and the equipment and an order for the movements of at least one of the group of the staff and the equipment.

In an example, not shown in detail, as further input, the data input 12 is configured to provide radiation dose information for at least a part of the staff members. Based on the respective location, the data processor 14 is configured to compute a dose exposure probability and to determine an adjusted position information to reduce radiation exposure of the staff.

As an example, staff members, e.g. doctors and nurses, working in a Cathlab experience radiation on a daily basis. Wearing dosimeters that measure the radiation input over time, respective radiation dose exposure is made available. This data is used as input to reduce the radiation input. For this purpose, tracking the procedure type and specifics along a timeline is provided. The dosimeters track the radiation intake at a specific moment during the procedure. Further, location tracking of each individual is provided, e.g. by Wi-Fi localization, Bluetooth beacon localization, computer vision systems, 3D cameras and the like. By providing a correlating timeline, radiation intake and location can be used to map where most radiation is likely to occur during a procedure. This data is used to improve the lab setup, e.g. staff and equipment positioning, for specific procedures to minimize radiation intake. In an option, this can be done as a static setup or even be enhanced to move equipment, and also staff, to radiation blank spots during procedure.

In an example, for determining the equipment instructions and the staff spatial guidance, the data processor 14 is configured to provide a parameter weighting to achieve an overall optimal score.

In an example, for determining the equipment instructions and the staff spatial guidance, a weighting for a plurality of the movements is provided to determine an optimal scoring across the plurality of the movements for predetermined parameter scores comprising at least one of the group of: target position in view of a targeted task, path length, path trajectory, intersecting with other trajectories, point of time of movement, speed of movement and continued necessary connection to supply lines or conduits.

In an example, for calculating the possible upcoming target positions, a weighting as in the example above is provided for a plurality of the movements to determine an optimal scoring across the plurality of the movements for the predetermined parameter scores.

In an example, for calculating the from-to routes, a weighting as in the example above is provided for a plurality of the movements to determine an optimal scoring across the plurality of the movements for the predetermined parameter scores.

In an example, for calculating the order for the movements, a weighting as in the example above is provided for a plurality of the movements to determine an optimal scoring across the plurality of the movements for the predetermined parameter scores.

In an example, the data processor 14 is configured to generate a specific procedure room layout configuration based on a plurality of generic room layout configurations. The data processor 14 is also configured to apply the generic room layout configurations to a model of a specific operation room stored in an operation room model store.

In a first option of a further example, the data processor 14 is configured to detect, based on changes and/or input by the user, deviations from at least one of the group of the provided equipment movement instructions and the staff spatial guidance. The data processor 14 is also configured to propose changes to stored operation room settings to the user providing a learning update.

In a second option of the further example, provided in addition or alternatively, the data processor 14 is configured to detect, based on tracked patient vital parameters, changes above a predetermined threshold indicating a possible upcoming emergency scenario. The data processor 14 is also configured to provide emergency equipment movement instructions and emergency staff spatial guidance.

An example for tracked patient vital parameters is the so-called hemo-stability index.

In an example, motorized wheels are provided for the drivable base, e.g. for mobile equipment. In another example, an actuatable boom is provided for mounting of imaging devices, like cameras, or lighting devices. An actuatable boom can also be provided for arranging patient supply and monitoring devices during operation. In a further example, a pan-tilt mechanism is provided for displays. At least a part of the equipment is configured to be moved automatically according to the movement instructions.

In an example, procedure data is provided before a procedure is started and the available equipment is tracked. Based on equipment movement instructions and staff spatial guidance, a setting up of the operation room or other room type for the upcoming procedure is provided. Further, as the procedure is started and is going on, the procedure data is provided at least in regular intervals, or even continuously, and the equipment and staff are re-positioned based on updated equipment movement instructions and staff spatial guidance.

As an effect, the staff no longer has to manually set up the operation room for the upcoming procedure and reposition the equipment as the procedure commences.

In an example, experienced operation room teams have learned what setups work well for certain procedures, and procedure phases, possibly in relation to physician preferences. As an option, these are documented. The knowledge is provided to set up the room. Due to the updated equipment movement instructions and staff spatial guidance, layout changes no longer require manual labor.

The operation room equipment, for example on motorized wheels, is moved to optimal positions and orientations dependent on the (anticipated) procedure phase, together with guidance to the involved staff, and with the necessary safeguard mechanisms. The anticipatory nature of the solution guarantees that equipment and people are in optimal positions for the tasks at hand, and avoids that staff needs to fetch, reposition and/or reorient (heavy) equipment manually.

For example, this is applicable for movable operation room equipment on motorized wheels, with actuatable booms or pan-tilt mechanisms. Optimal positions and orientations dependent on the (anticipated) procedure phase are achieved, together with guidance to the involved staff, and with the necessary safeguard mechanisms.

As an effect, orchestrated motorized equipment movement in an operation room is provided.

According to an example, not shown in further details, the data-processor 14 is configured to calculate, based on the provide procedure data, the spatial data and the calculated upcoming physical movement, alternative positions under the doctrine that at least a part of the group of staff and equipment is organized according to a flock-of-birds concept. The data-processor 14 is further configured to assess if any of the alternative positions provides benefits for upcoming stages. The data-processor 14 is also configured to provide at least one of the group of adjusted equipment movement instructions and adjusted staff spatial guidance.

In an example, several devices are provided. At least a part of the movable participants comprising staff and equipment is assigned with an individual device such that these can optimize their position under the flock-of-birds concept, also known as flocking. The (moving) participants achieve optimized positions regarding the actual moving forward in the context of a plurality of neighboring moving participants.

Fig. 2 shows an example of a system 100 for guidance in spatial setups of an operation room OR, or other type of room, in a medical facility. The system 100 comprises an example of the device 10 according to one of the preceding examples and options. The system 100 also comprises a capturing arrangement 102. The capturing arrangement 102 is configured to capture a current executed medical procedure and to detect spatial location and arrangement of at least one of the group of staff and equipment. The device 10 for guidance is configured to determine the procedure data based on the captured current executed medical procedure. The device 10 for guidance is also configured to determine the spatial data of the staff and/or equipment based on the detected spatial location and arrangement of at least one of the group of staff and equipment.

The term "capturing arrangement" relates to a device, system or other setup that is capable of capturing a current scene and to detect what is currently going on.

In an example, a system is provided comprising: a staff and equipment position and orientation tracker, a procedure type and phase tracker, (optionally) a procedure room layout configuration store, and a staff and equipment routing planner. The staff and equipment position and orientation tracker provides tracking data to the procedure type and phase tracker. The staff and equipment routing planner receives data from the staff and equipment position and orientation tracker and the procedure type and phase tracker. The staff and equipment routing planner (optionally) receives data from the procedure room layout configuration store. Further, the staff and equipment routing planner is connected with an equipment movement direction control and a staff guidance user interface.

In an option, an operation room model store and a generic procedure room layout configuration store are provided. They provide data to a procedure room layout configuration generator, which provides data to the procedure room layout configuration store.

In a further option, provided in addition, a procedure room layout optimizer / customizer is provided that receives data from the staff and equipment position and orientation tracker and the procedure type and phase tracker. The procedure room layout optimizer / customizer is further data-connected with the procedure room layout configuration generator and the generic procedure room layout configuration store.

In a still further option, provided in addition, an adaptive room layout controller is provided that receives data from the staff and equipment position and orientation tracker and that is connected with the equipment movement direction control.

In an example, the capturing arrangement 102 comprises at least one of the group of optical cameras, time of flight cameras, radar sensors, microphones and position and orientation tracking devices.

In an example, the system 100 further comprises equipment. At least a part of the equipment is provided with at least one of the group of: i) a drivable base, wherein the equipment movement instructions are provided to operate the base to move the equipment accordingly; ii) an actuatable boom, wherein the equipment movement instructions are provided to actuate the boom to reposition the equipment accordingly; and iii) an actuatable pan-tilt mechanism. The equipment movement instructions are provided to actuate the pan-tilt mechanism to adjust the equipment accordingly. At least a part of the equipment is configured to be moved automatically according to the movement instructions.

In an example, at least part of the staff and the equipment as participants in a procedure is organized under a flock-of-birds concept, according to which at least two of the participants, comprising at least two members of a group comprising at least one of the staff member and the equipment, are provided with its own algorithm running on a processor to optimize its position in the flock
In Fig. 2, as options, an optical camera 104, a time of flight cameras arrangement 106 and a microphone 108 are schematically shown.

As an example for one of many stages in an operation room OR, a first staff member 112, like a surgeon, and a second staff member 114, like an assisting person, are shown. A subject 116 is arranged on a subject support 118. Further, equipment like a mobile base 120 with light and displays is indicated. Still further, a mobile C-arm 122 is provided for imaging purposes.

The mobile base 120 with light and displays is provided with a first drivable base 124. The mobile C-arm 122 is provided with a second drivable base 126. Upon receiving respective signals such as the equipment movement instructions 22, the bases can move the equipment to a respective target location.

As a further option, a staff guidance instructor 128 is indicated. The staff guidance instructor 128 can also be referred to as staff guiding device. The staff guidance instructor 128 is configured to provide the staff spatial guidance 24 to at least one of the staff members 112, 114. The staff spatial guidance 24 can be provided, as an example, in form of a projection on the floor such that the respective staff member knows the target position.

In another option, not shown in detail, the staff spatial guidance 24 is provided via head-mounted devices, e.g. virtual or augmented reality devices.

In a further option, not shown, the staff spatial guidance 24 is provided via loudspeakers. To avoid any unnecessary noise within the operation room OR, a plurality of loudspeakers is provided to provide the staff spatial guidance 24 as sound-based information in a targeted, i.e. focused way. In an option, the staff spatial guidance 24 is provided in a way that the respective staff member is provided primarily, but also other staff members in the close surrounding are provided with this information such that they are also aware of the guidance. In an option, the staff spatial guidance 24 is provided via earphones.

In an example, not further shown in detail in the figures, at least part of the staff and the equipment as participants in a procedure is organized under a flock-of-birds concept, according to which at least two of the participants, comprising at least two members of a group comprising at least one of the staff member and the equipment, are provided with their own algorithm running on a processor to optimize its position in the flock.

For example, this provides a decentralized approach where every device and staff member digital twin have rules, e.g. learned rules to take optimal positions in procedure steps. As an example, certain devices may try to be always in reach of certain staff members.

In an aspect, a so-to-speak flock of operation room equipment amongst a so-to-speak flock of operation room staff is monitored, while tracking the progression of an operation room procedure. The flocks are driven via predefined procedural room layout configurations, in anticipation of the next procedural step or phase.

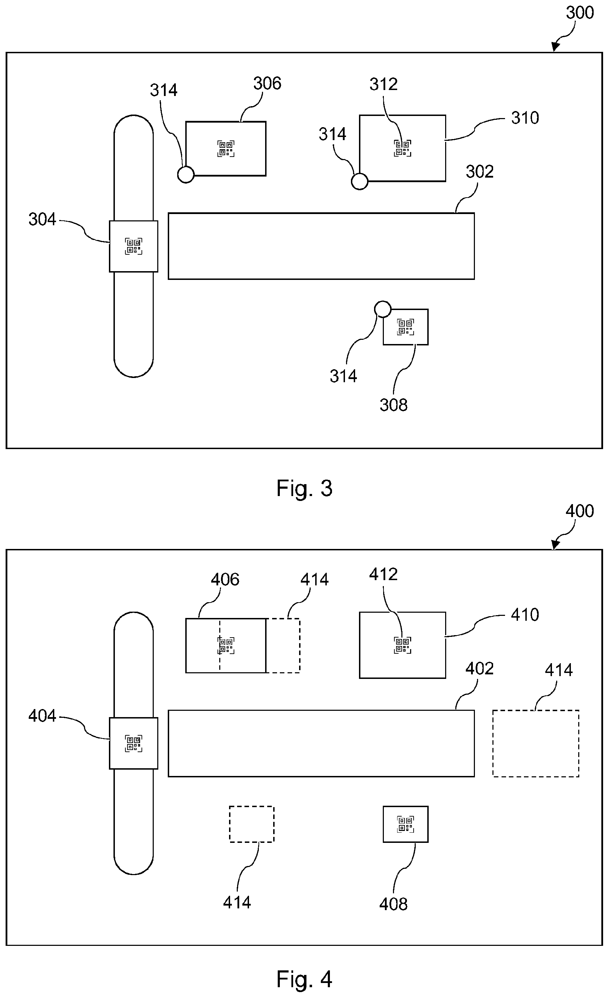

Fig. 3 shows another example of the system for guidance where a part of the equipment is provided with a motorized base. Fig. 3 shows a first schematic plan view 300. A subject support 302 is indicated together with a X-arm type X-ray imaging device 304 on the left side. The X-ray imaging device 304 can be provided with a mobile base or movably attached to a fixed support, such as a ceiling mounted rail system. Further, an anaesthesia machine 306, e.g. a base for providing anaesthesia and monitoring the subject's vital data, and an ultrasound machine 308 are indicated on both sides of the subject support 302. Further equipment 310 can also be arranged in the operation room, in particular near the subject support 302.

In an option, tracking markers 312 are provided for at least a part of the devices, e.g. attached to the anaesthesia machine 306, the ultrasound machine 308 or the further equipment 310.

The tracking markers 312 are provided for providing the spatial data 20 of the respective equipment. In addition, also staff members can be provided with tracking markers 312 or the like.

In order to accomplish the equipment movement instructions 22, at least a part of the equipment is having a drive mechanism 314, also referred as drivable base, to move the respective equipment accordingly. Instead of or in addition to the drive mechanism 314, an actuable boom or actuable pan-tilt mechanism is provided.

Fig. 4 shows a further example of the system for guidance with indicated projections or target positions for a part of the equipment. Fig. 4 shows a second schematic plan view 400, similar to Fig. 3. A subject support 402 is indicated together with a X-arm type X-ray imaging device 404 on the left side. Further, an anaesthesia machine 406, an ultrasound machine 408 and further equipment 410 are indicated on both sides of the subject support 402. Again as an option, tracking markers 412 are provided for at least a part of the devices.

In order to accomplish the equipment movement instructions 22 for at least a part of the equipment, projections 414 are provided indicating target positions. The respective equipment can then be moved accordingly, e.g. motorized driven, such as via remote control, or also manually.

In an example, equipment tracking and providing of auto recommendations for spatial arrangement is provided, for example as an application for, i.e. during, interventional procedures.

This provides particular advantages, since equipment within the cathlab is cumbersome and takes up a lot of already limited space. The guidance in spatial setups of an operation room facilitates the workflow. This is of advantage, since - with machines, cables, monitors, the patient bed and the moving C-arm, along with people in the room - coordination of the space is difficult. The provided solution supports maintaining a level of comfort and safety within the OR. To create an effective room setup, the device for guidance in spatial setups of an operation room understands at least the primary procedure requirements and sets the room accordingly based on point-in-time procedure requirements and potential equipment conflicts.

For providing the staff spatial guidance 24, but also the equipment movement instructions 22, annotating 2D images or 3D volumes in mixed reality is provided. The setup can also be used for staff training purposes.

To streamline the equipment maneuvering process, automated equipment tracking is provided to detect object location and potential collisions based on e.g. C-arm manipulations, or other machinery movements within the operation room. An option utilizes a marker-based system, using e.g. ceiling mounted cameras to locate objects over a 2D plane in the room. Based on calculations, predictions can be made to detect when a collision or obstruction might occur. Using an alerting system, such as lights on equipment within the room, users can be notified when a collision may occur, and adjustments can be made based on recommendations.

In another option, projection-based room recommendations are provided, whereby suggested recommended machine movements are projected into the room, such as on the floor, to navigate a technician to manually maneuver equipment to an ideal location.

In an aspect, sensor-based equipment tracking with prediction feedback for quick room setup and inter procedural equipment maneuvering is provided.

In a further option, provided in addition or alternatively and not shown in detail, the positioning recommendations are projected such that they are visible to staff members, but the movement is done automatically by controlling respective drives and actuators. By the projections, staff members are visually informed where a moving part is going to move to. In an option, this is provided in addition to projections of instructions for the staff members. In another option, the projections for the motorized moving equipment are provided without projections of instructions for the staff members. The latter might be provided as acoustic instructions.

In another option, provided in addition or alternatively and not shown in detail, projection-based staff positioning recommendations are provided, whereby suggested recommended staff movements are projected into the room, such as on the floor, to navigate staff to move themselves to an ideal location. In another aspect, sensor-based staff tracking with prediction feedback for quick room setup and inter procedural equipment maneuvering is also provided.

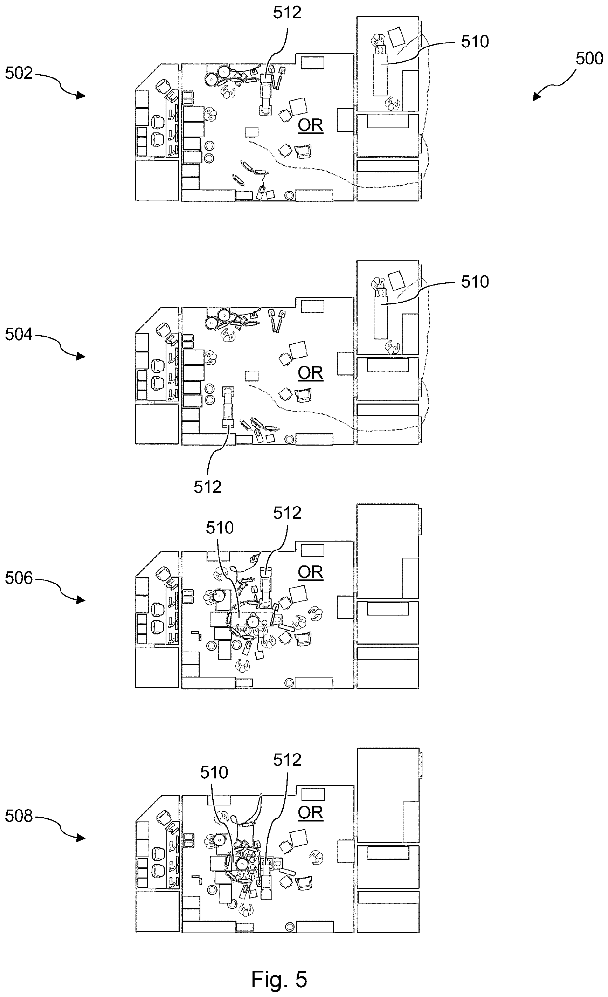

Fig. 5 shows four different scenarios or stages during a procedure 500 in an operation room. A first stage 502 is shown in the first row, a second stage 504 in the second row, a third stage 506 in the third row and a fourth stage 508 in the fourth row. The stages are exemplarily only, and many more different stages may exist during medical procedures.

In the first stage 502, a starting or preparation phase is shown. A subject support 510 is arranged in a preparation room. An X-ray imaging device 512, provided as a mobile or at least movable C-arm system, is in a parked position. In the second stage 504, the X-ray imaging device 512 is in a stand-by position for use. The subject support 510 is about to be moved inside the operation room. In the third stage 506, the subject support 510 is in an operation place and the X-ray imaging device 512 is in a first use position where it can be used for imaging a subject on the subject support 510. Further equipment and staff members are shown in various positions around the subject support 510. In the fourth stage 508, the X-ray imaging device 512 is in a second use position where it can be used for further imaging the subject on the subject support 510.

As an example for different stages during operations, it is referred to TAVI procedures, where trans apical stages differ from trans femoral stages.

The guidance in spatial setups of an operation room as provided by the various examples and options herewith help in organizing and handling the various movable parts, i.e. equipment, devices, systems, and also staff members and thus facilitate the complex workflow.

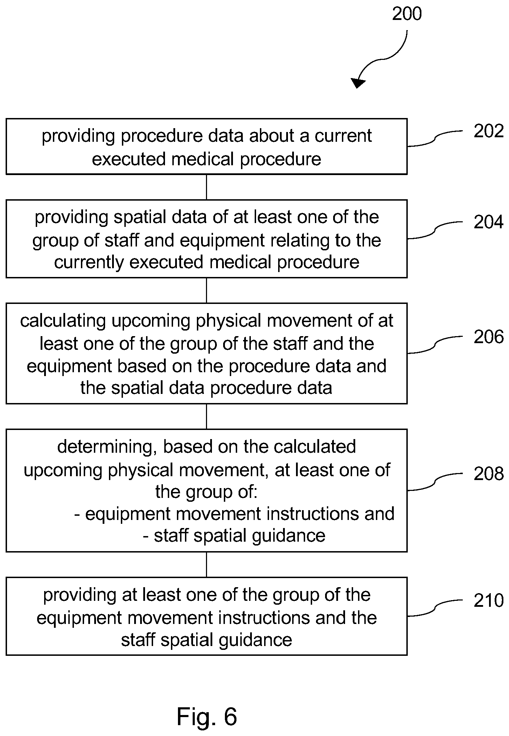

Fig. 6 shows basic steps of an example of a method 200 for guidance in spatial setups of a room in a medical facility. The method 200 comprises the following steps: In a first step 202, procedure data about a current executed medical procedure is provided. In a second step 204, spatial data of at least one of the group of staff and equipment relating to the currently executed medical procedure is provided. In a third step 206, upcoming physical movement of at least one of the group of the staff and the equipment is calculated based on the procedure data and the spatial data procedure data. In a fourth step 208, it is determined, based on the calculated upcoming physical movement, at least one of the group of: equipment movement instructions and staff spatial guidance. In a fifth step 210, at least one of the group of the equipment movement instructions and the staff spatial guidance is provided.

In an example of the method, for the providing of the procedure data about the current executed medical procedure, it is provided the step of tracking type and phase of the current executed medical procedure.

In an example of the method, for the tracking of the type and phase of the current executed medical procedure, it is provided at least one of the group of procedure analysis based on image data of a current scenario, procedure analysis based on acoustic data comprising at least parts of a conversation by the staff of a current scenario, and patient related data.

In an example of the method, for the providing of the spatial data, it is provided a step of optical tracking of physical objects in a predetermined space of the room based on optical image data.

In another example of the method, for the providing of the spatial data, it is provided a step of marker tracking of at least one of the group of the staff and the equipment. The markers are provided as at least one of the group of: optical markers, electromagnetic markers and acoustic markers. In an example, the acoustic markers are provided as ultrasound beacons.

In an example of the method, for the calculating of the upcoming physical movement, possible upcoming target positions of at least one of the group of the staff and the equipment are determined based on the procedure data and the spatial data procedure data. Further, possible movement trajectories are calculated; and a plausibility check is provided to select a target position and a trajectory.

In an example of the method, the calculating of the upcoming physical movement comprises calculating from-to routes for at least one of the group of the staff and the equipment.

In another example of the method, the calculating of the upcoming physical movement comprises calculating an order for the movements of at least one of the group of the staff and the equipment.

In an example of the method, for determining the equipment instructions and the staff spatial guidance, a parameter weighting is provided to achieve an overall optimal score.

In an example of the method, at least a part of the equipment is provided with a drivable base. The equipment movement instructions are provided to operate the base to move the equipment accordingly.

In an example of the method, at least a part of the equipment is provided with an actuatable boom. The equipment movement instructions are provided to actuate the boom to reposition the equipment accordingly.

In an example of the method, at least a part of the equipment is provided with an actuatable pan-tilt mechanism. The equipment movement instructions are provided to actuate the pan-tilt mechanism to adjust the equipment accordingly.

In an example of the method, a specific procedure room layout configuration is generated based on a plurality of generic room layout configurations. The generic room layout configurations are applied to a model of a specific operation room stored in an operation room model store.

In an example of the method, based on changes and/or input by the user, deviations from at least one of the group of the provided equipment movement instructions and the staff spatial guidance are detected. Further, changes to stored operation room settings are proposed to the user providing a learning update.

In an example of the method, based on tracked patient vital parameters, changes above a predetermined threshold are detected indicating a possible upcoming emergency scenario; and wherein emergency equipment movement instructions and emergency staff spatial guidance are provided.

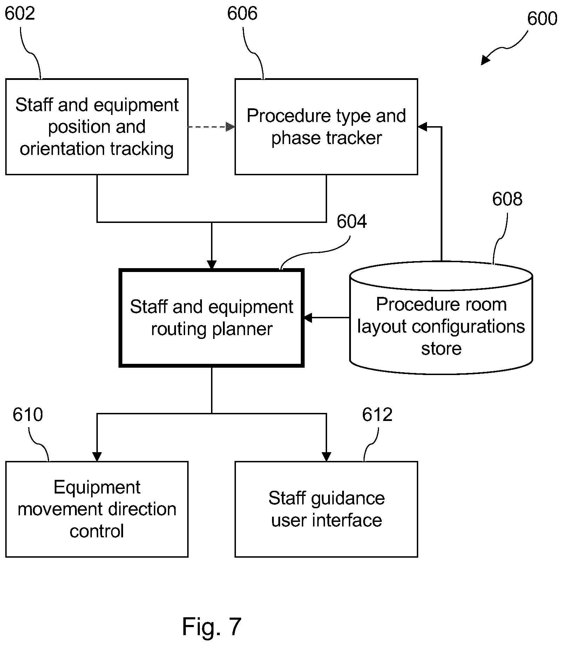

In the following, different examples and options are provided. These are also further explained below in exemplary setups as shown in Figs. 7 to 10.

In an example, a procedure type and phase tracker are provided. This may comprise to track what type of and phase within an operation room procedure is about to be executed. This information may come from the patient's electronic medical record, procedure cards, or procedure, e.g. phase, selection in an imaging system, ongoing procedure administration, and/or camera(s), e.g. ceiling mounted cameras like 3D cameras, with computer vision observing the procedure being executed, and or natural language processing analysis of the conversations in the operation room.

In an example, as an option, a procedure room layout configurations store is provided. This may comprise stored collection of room layouts, showing the optimal locations and orientations of equipment and staff per type and phase of a procedure.

In an example, a staff and equipment position and orientation tracking are provided. This may comprise trackers to track the position and orientation of the operation room staff and equipment in the room are provided. This may be done via, e.g. 3D camera(s), like ceiling mounted cameras, e.g. 3D cameras, with computer vision tracking the devices and people, possibly using posture, head orientation and/or eye-gaze tracking for the staff, or by using fiducial markers on the equipment, coded light, or object recognition algorithms, by Wi-Fi localization, optical floor pattern recognition, or via RFID / beacon-based localization, and the like.

In an example, a staff and equipment routing planner is provided. This may comprise an algorithm which calculates, e.g. continuously, from-to routes per equipment, but also per staff members, and the order in which these routes should be travelled, e.g. safely, without introducing collisions, by the staff and equipment. In an example, these routes go from the current to the next procedure room layout configuration, initiated by the procedure type and phase tracker.

In an example, a staff guidance user interface is provided: This may comprise one or several devices showing to the operation room staff members that equipment is moving or is about to start moving. This may also comprise showing where the staff members ideally should move themselves to, or where not to, for the time being to avoid collisions. In an example, this is done via projections on the floor by or visual and/or auditory signals on the equipment, spoken instructions, wearable augmented reality glasses, or the like. In an example, the guidance is driven by the staff and equipment routing planner.

In an example, an equipment movement direction control is provided: Instructions to the motorized wheels, booms, or pan-tilt mechanisms of each equipment are provided to reposition and/or reorient according to the route/direction provided by the staff and equipment routing planner.

It is proposed to rearrange operation room staff and operation room equipment, e.g. motorized wheels, motorized booms or motorized pan-tilt mechanisms, to accommodate for the ideal or optimized setup for type and phase of the operation room procedure at hand.

In a further option, a system is provided that learns from the execution of actual procedures on what room layouts, e.g. staff and equipment positions and orientations, per phase are most effective for a certain operation room team, which may be different per country, per hospital, depending on the type of equipment used and the number of staff involved, and the level of experience of the team. A procedure room layout optimizer, also referred to as a customizer component, is provided that uses input from the staff and equipment position and orientation tracking, as well as the procedure type and phase tracker to compare this with the information in the procedure room layout configurations store. If there is a consistent difference between actual and stored room layouts, the system proposes to update the related information in the procedure room layout configurations store. If the difference is consistently noticed in procedures in multiple operation rooms, hospitals and/or countries, the system even suggests updating the related info in the generic procedure room layout configurations store. This results in optimized and customized room layout configurations.

In an option, adaptive room layout generation is provided. As an example, staff members change position and orientation during a procedure phase, which is tracked. The equipment movement instructions and the staff spatial guidance are adapted to the situation as it happens in that moment. For example, adjusting positions and orientations of equipment enables the best ergonomics possible. An example for adapted room layout is shared display use.

In a further option, an adaptive room layout controller continuously uses the input from the staff and equipment position and orientation tracking, to drive the equipment movement direction control to generate small adjustments in equipment positions and orientations. For example, in the case of a shared display use, it may continuously analyze who is looking at what display, and accordingly adapt the viewing angle to the display such that all users looking at it have the best view possible, together.

For example, a staff and equipment position and orientation tracker is provided, to monitor, e.g. continuously, current position and orientation of all people and equipment in the operation room.

For example, a procedure type and phase tracker provided, to determine the start and end of a procedure, what type of procedure it is, and continuously monitors if a new phase in this procedure is about to be started for which different staff and equipment positions would be desired. For detecting phase transitions in the procedure, the system may use the staff and equipment position and orientation tracking, or other sources. It may also reference the procedure room layout configurations store to determine if position changes are to be made.

For example, a staff and equipment routing planner receives triggers from the procedure type and phase tracker that a new phase with position changes is about to start. In response to such a trigger it collects the newly desired staff and equipment positions from the procedure room layout configurations store and starts calculating displacement routes and/or new orientations for each staff member and piece of equipment, as well as how these movements are best orchestrated, and e.g. possibly partially sequenced, with best efficiency and while avoiding collisions. During the transition to new positions, it continuously monitors progress and may dynamically adapt routing strategies, if for example the staff makes other movements in relation to the guidance provided to them.

For example, a staff guidance user interface and an equipment movement direction control simultaneously get information from the staff and equipment routing planner to instruct each staff member on where to stand, position or orient themselves towards, in safe interplay with the equipment movement that takes place. The user interface may include notices that nearby equipment is about to start moving or has arrived at its final destination, possibly displayed on the equipment itself.

The from-to routes can also be referred to as current-location-to-target-location routes or movement paths.

In an option, generative room layout configurations are provided. For example, instead of having procedure room layout configurations per specific operation room or other room type for medical interventions and examinations in a specific hospital, the system generates specific procedure room layout configurations based on generic procedure room layout configurations. A procedure room layout configuration generator is provided which calculates specific procedure room layout configurations by applying generic specific procedure room layout configurations to a model of a specific operation room in a specific hospital, or other structure, stored in an operation room model store.

In the following, the term "supplying data" is used for describing the provision of data, of a signal or of any other form of information to the next part of the setup, i.e. the workflow diagram.

Fig. 7 shows a schematic setup and data workflow diagram 600 of an example for guidance in spatial setups of a room in a medical facility.

In Fig. 7, a staff and equipment position and orientation tracking 602 is provided that supplies data to a staff and equipment routing planner 604. Further, a procedure type and phase tracker 606 is provided that also supplies data to the staff and equipment routing planner 604. In addition, a procedure room layout configurations store 608 is provided in an option to provide data to the procedure type and phase tracker 606 and the staff and equipment routing planner 604. The respective data is processed in the staff and equipment routing planner 604 and then provided to an equipment movement direction control 610 and a staff guidance user interface 612. The equipment movement direction control 610, as a first output, handles the spatial placement, i.e. controls the arrangement of the equipment. The staff guidance user interface 612, as a second output, provides instructions, i.e. guidance for the staff.

As an option, the equipment position and orientation tracking 602 supplies data to the procedure type and phase tracker 606.

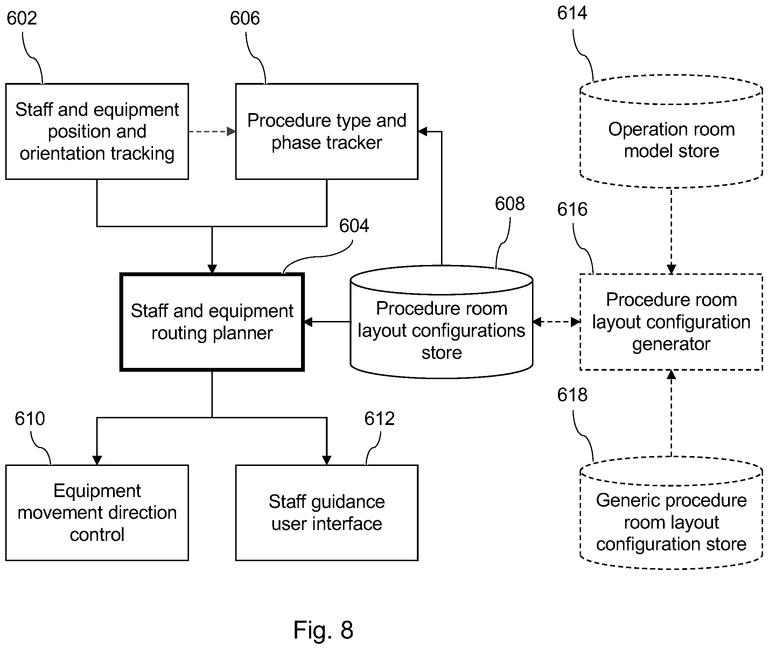

Fig. 8 shows an option for the setup and data workflow of Fig. 7.

In Fig. 8, an operation room model store 614 is provided that supplies data to a procedure room layout configuration generator 616. Further, also a generic procedure room layout configuration store 618 is provided that supplies data to the procedure room layout configurations store 608. Hence, the operation room model data and the generic procedure room layout configuration data can be used for the above-described data processing.

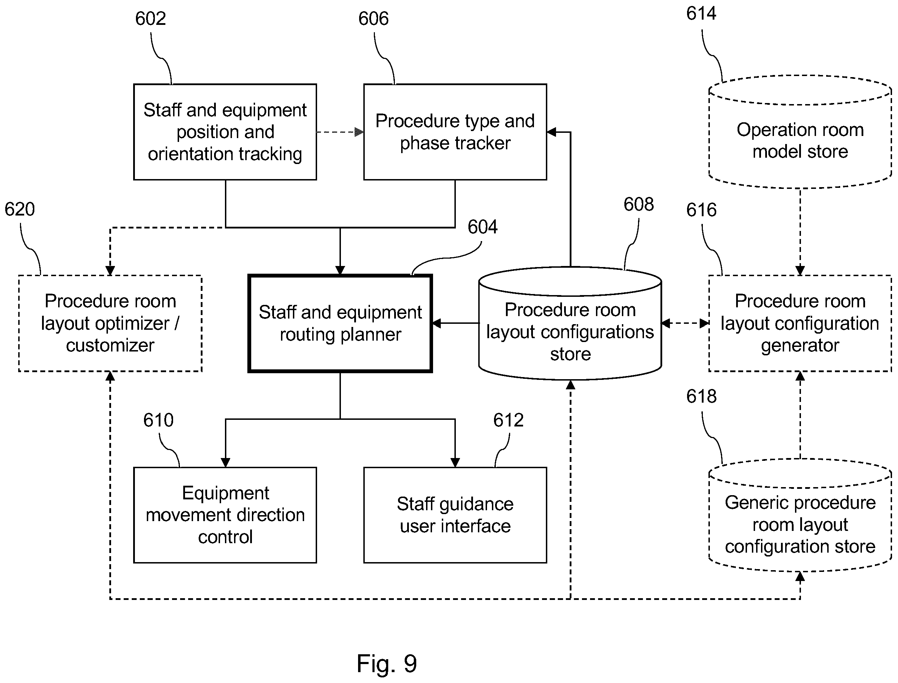

Fig. 9 shows further options for the setup and data workflow of Fig. 7. In a first option, the following is provided together with the option provided in Fig. 8. In a second option, the following is provided without the option provided in Fig. 8.

In Fig. 9, a procedure room layout optimizer/customizer 620 is provided that receives data from the staff and equipment position and orientation tracking 602 and/or the procedure type and phase tracker 606. The procedure room layout optimizer/customizer 620 supplies data to and receives data from the procedure room layout configurations store 608. In an option, together with the option shown in Fig. 8, the procedure room layout optimizer/customizer 620 supplies data to and receives data from the generic procedure room layout configuration store 618.

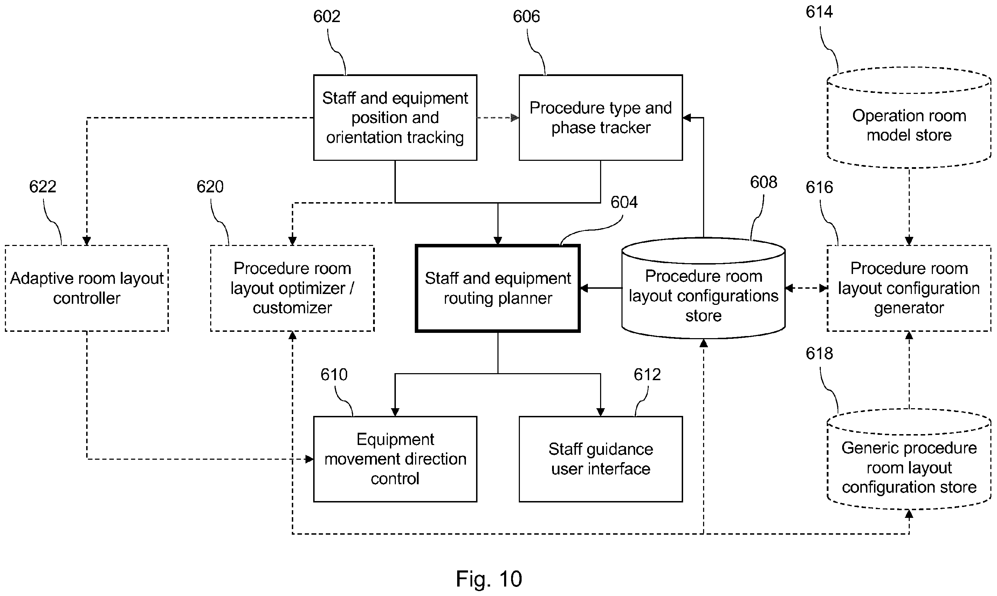

Fig. 10 shows still further options for the setup and data workflow of Fig. 7. In a first option, the following is provided together with the option provided in Fig. 8 and together with the option provided in Fig. 9. In a second option, the following is provided together with the option provided in Fig. 8, but without the option provided in Fig. 9. In a third option, the following is provided together with the option provided in Fig. 9, but without the option provided in Fig. 8. In a fourth option, the following is provided without the option provided in Fig. 8 and without the option provided in Fig. 9.

In Fig. 10, an adaptive room layout controller 622 is provided that supplies data to the equipment movement direction control 610. The adaptive room layout controller 622 uses the input from the staff and equipment position and orientation tracking to drive the equipment movement direction control to generate small adjustments in equipment positions and orientations.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for guidance in spatial setups of a room in a medical facility, the device comprising:
- a data input (12);
- a data processor (14); and
- an output interface (16);
wherein the data input is configured: to provide procedure data (18) about a current executed medical procedure; and to provide spatial data (20) of at least one of the group of staff and equipment relating to the currently executed medical procedure;
wherein the data processor is configured: to calculate upcoming physical movement of at least one of the group of the staff and the equipment based on the procedure data and the spatial data procedure data; and to determine, based on the calculated upcoming physical movement, at least one of the group of equipment movement instructions (22) and staff spatial guidance (24); and
wherein the output interface is configured: to provide at least one of the group of the equipment movement instructions and the staff spatial guidance;
wherein the upcoming physical movement relates to what will happen within the current scenario as a next action regarding the movement of the devices or persons;
wherein, for the provision of the procedure data about the current executed medical procedure, the data processor is configured to track type and phase of the current executed medical procedure; wherein, for the tracking of the type and phase of the current executed medical procedure, the data processor is configured to provide at least one of the group of: procedure analysis based on image data of a current scenario; procedure analysis based on acoustic data comprising at least parts of a conversation by the staff of a current scenario; and patient related data; and
wherein, for the provision of the spatial data, the data processor is configured to provide at least one of the following group of: optical tracking of physical objects in a predetermined space of the room based on optical image data; and marker tracking of at least one of the group of the staff and the equipment, wherein the markers are provided as at least one of the group of optical markers, electromagnetic markers and acoustic markers.

2. Device according to claim 1, wherein, for the calculation of the upcoming physical movement, the data processor is configured to determine possible upcoming target positions of at least one of the group of the staff and the equipment based on the procedure data and the spatial data; to calculate possible movement trajectories; and to provide a plausibility check to select a target position and a trajectory; and
wherein the data processor is configured to calculate the upcoming physical movement comprising at least one of the group of from-to routes for at least one of the group of the staff and the equipment and an order for the movements of at least one of the group of the staff and the equipment.

3. Device according to one of the preceding claims, wherein, as further input, the data input is configured to provide radiation dose information for at least a part of the staff members, and wherein, based on the respective location, the data processor is configured to compute a dose exposure probability and to determine an adjusted position information to reduce radiation exposure of the staff.

4. Device according to one of the preceding claims, wherein, for determining the equipment instructions and the staff spatial guidance, the data processor is configured to provide a parameter weighting to achieve an overall optimal score.

5. Device according to one of the preceding claims, wherein the data processor is configured to generate a specific procedure room layout configuration based on a plurality of generic room layout configurations; and to apply the generic room layout configurations to a model of a specific room stored in a room model store.

6. Device according to one of the preceding claims, wherein the data processor is configured to detect:
i) based on changes and/or input by the user, deviations from at least one of the group of the provided equipment movement instructions and the staff spatial guidance; and to propose changes to stored room settings to the user providing a learning update; and/or
ii) based on tracked patient vital parameters, changes above a predetermined threshold indicating a possible upcoming emergency scenario; and to provide emergency equipment movement instructions and emergency staff spatial guidance.

7. Device according to one of the preceding claims, wherein the data-processor is configured:
to calculate, based on the provide procedure data, the spatial data and the calculated upcoming physical movement, alternative positions under the doctrine that at least a part of the group of staff and equipment is organized according to a flock-of-birds concept; wherein, in the flock-of-birds concept, the moving participants achieve optimized positions regarding the actual moving forward in the context of a plurality of neighboring moving participants;
to assess if any of the alternative positions provides benefits for upcoming stages; and
to provide at least one of the group of adjusted equipment movement instructions and adjusted staff spatial guidance.

8. A system (100) for guidance in spatial setups of a room in a medical facility, the system comprising:
- a device (102) according to one of the preceding claims; and
- a capturing arrangement (104);
wherein the capturing arrangement is configured to capture a current executed medical procedure and to detect spatial location and arrangement of at least one of the group of staff and equipment; and
wherein the device for guidance is configured to determine the procedure data based on the captured current executed medical procedure; and to determine the spatial data of the staff and/or equipment based on the detected spatial location and arrangement of at least one of the group of staff and equipment.

9. System according to claim 8, wherein the capturing arrangement comprises at least one of the group of:
- optical cameras (104);
- time of flight cameras (106);
- radar sensors;
- microphones (108); and
- position and orientation tracking devices.

10. System according to claim 8 or 9, further comprising equipment; and
wherein at least a part of the equipment is provided with at least one of the group of:
i) a drivable base (124, 126; 314), wherein the equipment movement instructions are provided to operate the base to move the equipment accordingly;
ii) an actuatable boom, wherein the equipment movement instructions are provided to actuate the boom to reposition the equipment accordingly; and
iii) an actuatable pan-tilt mechanism, wherein the equipment movement instructions are provided to actuate the pan-tilt mechanism to adjust the equipment accordingly;
wherein at least a part of the equipment is configured to be moved automatically according to the movement instructions.

11. A method (200) for guidance in spatial setups of a room in a medical facility, comprising the following steps:
- providing (202) procedure data about a current executed medical procedure;
- providing (204) spatial data of at least one of the group of staff and equipment relating to the currently executed medical procedure;
- calculating (206) upcoming physical movement of at least one of the group of the staff and the equipment based on the procedure data and the spatial data procedure data;
- determining (208), based on the calculated upcoming physical movement, at least one of the group of:
- equipment movement instructions and
- staff spatial guidance; and
- providing (210) at least one of the group of the equipment movement instructions and the staff spatial guidance
wherein the upcoming physical movement relates to what will happen within the current scenario as a next action regarding the movement of the devices or persons;
wherein, for the provision of the procedure data about the current executed medical procedure. a data processor is configured to track type and phase of the current executed medical procedure; wherein, for the tracking of the type and phase of the current executed medical procedure, the data processor is configured to provide at least one of the group of: procedure analysis based on image data of a current scenario; procedure analysis based on acoustic data comprising at least parts of a conversation by the staff of a current scenario; and patient related data; and
wherein, for the provision of the spatial data, the data processor is configured to provide at least one of the following group of: optical tracking of physical objects in a predetermined space of the room based on optical image data; and marker tracking of at least one of the group of the staff and the equipment, wherein the markers are provided as at least one of the group of optical markers, electromagnetic markers and acoustic markers.

12. Computer program comprising instructions which, when the program is executed by a computer, cause the computer to carry out the method of claim 11.

## Patentansprüche

1. Vorrichtung (10) zur Führung in räumlichen Einstellungen eines Raumes in einer medizinischen Einrichtung, wobei die Vorrichtung umfasst:
- eine Dateneingabe (12);
- einen Datenprozessor (14); und
- eine Ausgabeschnittstelle (16);
wobei die Dateneingabe dazu konfiguriert ist, Eingriffsdaten (18) über einen aktuell ausgeführten medizinischen Eingriff bereitzustellen; und räumliche Daten (20) von zumindest einem aus der Gruppe von Personal und Geräten in Bezug auf den aktuell ausgeführten medizinischen Eingriff bereitzustellen;
wobei der Datenprozessor dazu konfiguriert ist, die bevorstehende physische Bewegung von zumindest einem aus der Gruppe von Personal und Geräten basierend auf den Eingriffsdaten und den räumlichen Daten Eingriffsdaten zu berechnen; und basierend auf der berechneten bevorstehenden physischen Bewegung zumindest eines aus der Gruppe von Gerätebewegungsanweisungen (22) und räumlicher Personalführung (24) zu bestimmen; und
wobei die Ausgabeschnittstelle dazu konfiguriert ist, zumindest eines aus der Gruppe von Gerätebewegungsanweisungen und der räumlichen Personalführung bereitzustellen;
wobei die bevorstehende physische Bewegung sich auf das bezieht, was im aktuellen Szenario als nächster Schritt in Bezug auf die Bewegung der Vorrichtungen oder Personen geschehen wird;
wobei der Datenprozessor zur Bereitstellung der Eingriffsdaten über den aktuell durchgeführten medizinischen Eingriff dazu konfiguriert ist, Typ und Phase des aktuell durchgeführten medizinischen Eingriffs zu verfolgen; wobei der Datenprozessor zum Verfolgen von Typ und Phase des aktuell ausgeführten medizinischen Eingriffs dazu konfiguriert ist, zumindest eines aus der Gruppe von Verfahrensanalyse basierend auf Bilddaten eines aktuellen Szenarios; Verfahrensanalyse basierend auf Akustikdaten, umfassend zumindest Teile eines Gesprächs des Personals eines aktuellen Szenarios; und patientenbezogenen Daten bereitzustellen; und
wobei der Datenprozessor zur Bereitstellung der räumlichen Daten dazu konfiguriert ist, zumindest eines aus der Gruppe von optischer Verfolgung physischer Objekte in einem vorbestimmten Bereich des Raumes basierend auf optischen Bilddaten; und Markerverfolgung von zumindest einem aus der Gruppe von Personal und Geräten bereitzustellen, wobei die Marker als zumindest eines aus der Gruppe von optischen Markern, elektromagnetischen Markern und akustischen Markern bereitgestellt werden.

2. Vorrichtung nach Anspruch 1, wobei der Datenprozessor zur Berechnung der bevorstehenden physischen Bewegung dazu konfiguriert ist, basierend auf den Eingriffsdaten und den räumlichen Daten mögliche bevorstehende Zielpositionen von zumindest einem aus der Gruppe von Personal und Geräten zu bestimmen; mögliche Bewegungstrajektorien zu berechnen; und eine Plausibilitätsprüfung bereitzustellen, um eine Zielposition und eine Trajektorie auszuwählen; und
wobei der Datenprozessor dazu konfiguriert ist, die bevorstehende physische Bewegung zu berechnen, welche zumindest eines aus der Gruppe von Von-Nach-Routen für zumindest eines aus der Gruppe von Personal und Geräten und einen Auftrag für die Bewegungen von zumindest einem aus der Gruppe von Personal und Geräten umfasst.

3. Vorrichtung nach einem der vorstehenden Ansprüche, wobei die Dateneingabe als weitere Eingabe dazu konfiguriert ist, Strahlendosisinformationen für zumindest einen Teil der Mitarbeiter bereitzustellen, und wobei der Datenprozessor basierend auf dem jeweiligen Standort dazu konfiguriert ist, eine Strahlenbelastungswahrscheinlichkeit zu berechnen und angepasste Positionsinformationen zu bestimmen, um die Strahlenbelastung des Personals zu reduzieren.

4. Vorrichtung nach einem der vorstehenden Ansprüche, wobei der Datenprozessor zum Bestimmen der Geräteanweisungen und der räumlichen Personalführung dazu konfiguriert ist, eine Parametergewichtung bereitzustellen, um eine optimale Gesamtpunktzahl zu erreichen.

5. Vorrichtung nach einem der vorstehenden Ansprüche, wobei der Datenprozessor dazu konfiguriert ist, eine spezifische Eingriffsraumgestaltungskonfiguration basierend auf einer Vielzahl von generischen Raumgestaltungskonfigurationen zu generieren; und die generischen Raumgestaltungskonfigurationen auf ein in einem Raummodellspeicher gespeichertes Modell eines spezifischen Raums anzuwenden.

6. Vorrichtung nach einem der vorstehenden Ansprüche, wobei der Datenprozessor dazu konfiguriert ist, folgendes zu erkennen:
i) basierend auf Änderungen und/oder Eingaben des Benutzers, Abweichungen von zumindest einem aus der Gruppe der bereitgestellten Gerätebewegungsanweisungen und der räumlichen Personalführung; und dem Benutzer Änderungen an gespeicherten Raumeinstellungen vorzuschlagen und ein Lernupdate bereitzustellen; und/oder
ii) basierend auf verfolgten Vitalparametern des Patienten, Änderungen oberhalb eines vorbestimmten Schwellenwerts, welche auf ein mögliches bevorstehendes Notfallszenario hinweisen; und Notfall-Gerätebewegungsanweisungen und räumliche Notfall-Personalführung bereitzustellen.

7. Vorrichtung nach einem der vorstehenden Ansprüche, wobei der Datenprozessor dazu konfiguriert ist:
basierend auf den bereitgestellten Eingriffsdaten, den räumlichen Daten und den berechneten bevorstehenden physischen Bewegung alternative Positionen, unter der Lehre zu berechnen, dass zumindest ein Teil der Gruppe von Personal und Geräten gemäß einem Vogelschwarm-Konzept organisiert ist; wobei im Vogelschwarm-Konzept die sich bewegenden Teilnehmer im Hinblick auf die tatsächliche Vorwärtsbewegung im Kontext einer Vielzahl von benachbarten sich bewegenden Teilnehmer optimierte Positionen erreichen;
um zu beurteilen, ob eine der alternativen Positionen Vorteile für die bevorstehende Phasen bereitstellt; und
zumindest eines aus der Gruppe von angepassten Gerätebewegungsanweisungen und der angepassten räumlichen Personalführung bereitzustellen.

8. System (100) zur Führung in räumlichen Einstellungen eines Raumes in einer medizinischen Einrichtung, wobei das System umfasst:
- eine Vorrichtung (102) nach einem der vorstehenden Ansprüche; und
- eine Erfassungsanordnung (104);
wobei die Erfassungsanordnung dazu konfiguriert ist, einen aktuell ausgeführten medizinischen Eingriff zu erfassen und die räumliche Position und Anordnung von zumindest einem aus der Gruppe von Personal und Geräten zu erkennen; und
wobei die Vorrichtung zur Führung dazu konfiguriert ist, die Eingriffsdaten basierend auf dem erfassten, aktuell durchgeführten medizinischen Eingriff zu bestimmen; und die räumlichen Daten des Personals und/oder der Geräte basierend auf der erkannten räumlichen Position und Anordnung von zumindest einem aus der Gruppe von Personal und Geräten zu bestimmen.

9. System nach Anspruch 8, wobei die Erfassungsanordnung zumindest eines aus der folgenden Gruppe umfasst:
- optische Kameras (104);
- Laufzeitkameras (106);
- Radarsensoren;
- Mikrofone (108); und
- Positions- und Orientierungsverfolgungsvorrichtungen.

10. System nach Anspruch 8 oder 9, weiter Geräte umfassend; und
wobei zumindest ein Teil der Geräte mit zumindest einem aus der folgenden Gruppe versehen ist:
i) einer fahrbaren Basis (124, 126; 314), wobei die Gerätebewegungsanweisungen bereitgestellt werden, um die Basis zu bedienen und die Geräte entsprechend zu bewegen;
ii) einem ansteuerbaren Ausleger, wobei die Gerätebewegungsanweisungen bereitgestellt werden, um den Ausleger zu betätigen, um die Geräte entsprechend neu zu positionieren; und
iii) einem ansteuerbaren Schwenk-Neige-Mechanismus, wobei die Gerätebewegungsanweisungen bereitgestellt werden, um den Schwenk-Neige-Mechanismus zu betätigen, um das Gerät entsprechend anzupassen;
wobei zumindest ein Teil der Geräte dazu konfiguriert ist, gemäß den Bewegungsanweisungen automatisch bewegt zu werden.

11. Verfahren (200) zur Führung in räumlichen Einstellungen eines Raumes in einer medizinischen Einrichtung, umfassend die folgenden Schritte:
- Bereitstellen (202) von Eingriffsdaten über einen aktuell durchgeführten medizinischen Eingriff;
- Bereitstellen (204) von räumlichen Daten von zumindest einem aus der Gruppe von Personal und Geräten in Bezug auf den aktuell ausgeführten medizinischen Eingriff;
- Berechnen (206) einer bevorstehenden physischen Bewegung von zumindest einem aus der Gruppe von Personal und Geräten basierend auf den Eingriffsdaten und den räumlichen Daten der Eingriffsdaten;
- basierend auf der berechneten bevorstehenden physischen Bewegung, Bestimmen (208) von zumindest einem aus der folgenden Gruppe:
- Gerätebewegungsanweisungen und
- räumliche Personalführung; und
- Bereitstellen (210) von zumindest einem aus der Gruppe von Gerätebewegungsanweisungen und der räumlichen Personalführung,
wobei die bevorstehende physische Bewegung sich auf das bezieht, was im aktuellen Szenario als nächster Schritt in Bezug auf die Bewegung der Vorrichtungen oder Personen geschehen wird;
wobei ein Datenprozessor zur Bereitstellung der Eingriffsdaten über den aktuell durchgeführten medizinischen Eingriff dazu konfiguriert ist, Typ und Phase des aktuell durchgeführten medizinischen Eingriffs zu verfolgen; wobei der Datenprozessor zum Verfolgen von Typ und Phase des aktuell ausgeführten medizinischen Eingriffs dazu konfiguriert ist, zumindest eines aus der Gruppe von Verfahrensanalyse basierend auf Bilddaten eines aktuellen Szenarios; Verfahrensanalyse basierend auf Akustikdaten, umfassend zumindest Teile eines Gesprächs des Personals eines aktuellen Szenarios; und patientenbezogenen Daten bereitzustellen; und
wobei der Datenprozessor zur Bereitstellung der räumlichen Daten dazu konfiguriert ist, zumindest eines aus der Gruppe von optischer Verfolgung physischer Objekte in einem vorbestimmten Bereich des Raumes basierend auf optischen Bilddaten; und Markerverfolgung von zumindest einem aus der Gruppe von Personal und Geräten bereitzustellen, wobei die Marker als zumindest eines aus der Gruppe von optischen Markern, elektromagnetischen Markern und akustischen Markern bereitgestellt werden.

12. Computerprogramm, welches Anweisungen umfasst, die, wenn das Programm von einem Computer ausgeführt wird, den Computer veranlassen, das Verfahren nach Anspruch 11 auszuführen.

## Revendications

1. Dispositif (10) destiné au guidage dans des configurations spatiales d'une pièce dans un établissement médical, le dispositif comprenant :
- une entrée de données (12) ;
- un processeur de données (14) ; et
- une interface de sortie (16) ;
dans lequel l'entrée de données est configurée : pour fournir des données d'intervention (18) relatives à rune intervention médicale en cours d'exécution ; et pour fournir des données spatiales (20) d'au moins un du groupe du personnel et de l'équipement se rapportant à l'intervention médicale en cours d'exécution ;
dans lequel le processeur de données est configuré : pour calculer un déplacement physique à venir d'au moins un du groupe du personnel et de l'équipement sur la base des données d'intervention et des données d'intervention de données spatiales ; et pour déterminer, sur la base du déplacement physique à venir calculé, au moins une du groupe d'instructions de déplacement d'équipement (22) et d'un guidage spatial de personnel (24) ; et
dans lequel l'interface de sortie est configurée : pour fournir au moins une du groupe des instructions de déplacement d'équipement et du guidage spatial de personnel ;
dans lequel le déplacement physique à venir se rapporte à ce qui se produira dans le scénario actuel en tant que prochaine action concernant le déplacement des dispositifs ou des personnes ;
dans lequel, pour la fourniture des données d'intervention relatives à l'intervention médicale en cours d'exécution, le processeur de données est configuré pour suivre le type et la phase de l'intervention médicale en cours d'exécution ; dans lequel, pour le suivi du type et de la phase de l'intervention médicale en cours d'exécution, le processeur de données est configuré pour fournir au moins un du groupe : analyse d'intervention sur la base de données d'image d'un scénario actuel ; analyse d'intervention sur la base de données acoustiques comprenant au moins des extraits d'une conversation du personnel d'un scénario actuel ; et données se rapportant au patient ; et
dans lequel, pour la fourniture des données spatiales, le processeur de données est configuré pour fournir au moins un du groupes suivant : suivi optique d'objets physiques dans un espace prédéterminé de la pièce sur la base de données d'image optique ; et suivi par marqueurs d'au moins un du groupe du personnel et de l'équipement, dans lequel les marqueurs sont fournis comme au moins un du groupe de marqueurs optiques, de marqueurs électromagnétiques et de marqueurs acoustiques.

2. Dispositif selon la revendication 1, dans lequel, pour le calcul du déplacement physique à venir, le processeur de données est configuré pour déterminer des positions cibles à venir possibles d'au moins un du groupe du personnel et de l'équipement sur la base des données d'intervention et des données spatiales ; pour calculer des trajectoires de déplacement possibles ; et pour fournir un contrôle de plausibilité pour sélectionner une position cible et une trajectoire ; et
dans lequel le processeur de données est configuré pour calculer le déplacement physique à venir comprenant au moins un du groupe des itinéraires de départ et d'arrivée pour au moins un du groupe du personnel et de l'équipement et un ordre pour les déplacements d'au moins un du groupe du personnel et de l'équipement.

3. Dispositif selon l'une des revendications précédentes, dans lequel, en tant qu'entrée supplémentaire, l'entrée de données est configurée pour fournir des informations sur la dose de rayonnement pour au moins une partie des membres du personnel, et dans lequel, sur la base de l'emplacement respectif, le processeur de données est configuré pour calculer une probabilité d'exposition à la dose et pour déterminer des informations de position ajustées pour réduire une exposition du personnel aux rayonnements.

4. Dispositif selon l'une des revendications précédentes, dans lequel, pour déterminer les instructions de l'équipement et le guidage spatial de personnel, le processeur de données est configuré pour fournir une pondération de paramètres pour obtenir un score optimal global.

5. Dispositif selon l'une des revendications précédentes, dans lequel le processeur de données est configuré pour générer une configuration d'aménagement de salle d'intervention spécifique sur la base d'une pluralité de configurations d'aménagement de salle génériques ; et pour appliquer les configurations d'aménagement de salle génériques à un modèle d'une salle spécifique stocké dans un référentiel de modèles de salles.

6. Dispositif selon l'une des revendications précédentes, dans lequel le processeur de données est configuré pour détecter :
i) sur la base de modifications et/ou d'une entrée par l'utilisateur, des écarts par rapport à au moins une du groupe des instructions de déplacement d'équipement fournies et du guidage spatial de personnel ; et pour proposer des modifications aux paramètres de salle enregistrés à l'utilisateur en fournissant une mise à jour sur l'apprentissage ; et/ou
ii) sur la base de paramètres vitaux de patient suivis, des modifications au-dessus d'un seuil prédéterminé indiquant un scénario d'urgence possible à venir ; et pour fournir des instructions de déplacement d'équipement d'urgence et un guidage spatial de personnel d'urgence.

7. Dispositif selon l'une des revendications précédentes, dans lequel le processeur de données est configuré :
pour calculer, sur la base des données d'intervention fournies, des données spatiales et du déplacement physique à venir calculé, des positions alternatives selon la doctrine selon laquelle au moins une partie du groupe du personnel et de l'équipement est organisée selon un concept de volée d'oiseaux ; dans lequel, dans le concept de volée d'oiseaux, les participants en déplacement atteignent des positions optimisées par rapport à la progression réelle dans le contexte d'une pluralité de participants en déplacement voisins ;
pour évaluer si l'une quelconque des positions alternatives fournit des avantages pour les étapes à venir ; et
pour fournir au moins une du groupe des instructions de déplacement d'équipement ajustées et du guidage spatial de personnel ajusté.

8. Système (100) destiné au guidage dans des configurations spatiales d'une pièce dans un établissement médical, le système comprenant :
- un dispositif (102) selon l'une des revendications précédentes ; et
- un agencement de capture (104) ;
dans lequel l'agencement de capture est configuré pour capturer une intervention médicale en cours d'exécution et pour détecter un emplacement spatial et un agencement d'au moins un du groupe du personnel et de l'équipement ;
dans lequel le dispositif destiné au guidage est configuré pour déterminer les données d'intervention sur la base de l'intervention médicale en cours d'exécution capturée ; et pour déterminer les données spatiales du personnel et/ou de l'équipement sur la base de l'emplacement spatial et de l'agencement détectés d'au moins un du groupe du personnel et de l'équipement.

9. Système selon la revendication 8, dans lequel l'agencement de capture comprend au moins un du groupe de :
- caméras optiques (104) ;
- caméras à temps de vol (106) ;
- capteurs radar ;
- microphones (108) ; et
- dispositifs de suivi de position et d'orientation.

10. Système selon la revendication 8 ou 9, comprenant en outre un équipement ; et
dans lequel au moins une partie de l'équipement est dotée d'au moins un du groupe :
i) une base motrice (124, 126 ; 314), dans laquelle les instructions de déplacement d'équipement sont fournies pour actionner la base pour déplacer l'équipement en conséquence ;
ii) une flèche actionnable, dans lequel les instructions de déplacement d'équipement sont fournies pour actionner la flèche pour repositionner l'équipement en conséquence ; et
iii) un mécanisme d'inclinaison panoramique actionnable, dans lequel les instructions de déplacement d'équipement sont fournies pour actionner le mécanisme d'inclinaison panoramique pour ajuster l'équipement en conséquence ;
dans lequel au moins une partie de l'équipement est configurée pour être déplacée automatiquement selon les instructions de déplacement.

11. Procédé (200) destiné au guidage dans des configurations spatiales d'une pièce dans un établissement médical, comprenant les étapes suivantes :
- fourniture (202) de données d'intervention relatives à une intervention médicale en cours d'exécution ;
- fourniture (204) de données spatiales d'au moins un du groupe du personnel et de l'équipement se rapportant à l'intervention médicale en cours d'exécution ;
- calcul (206) du déplacement physique à venir d'au moins un du groupe du personnel et de l'équipement sur la base des données d'intervention et des données d'intervention de données spatiales ;
- détermination (208), sur la base du déplacement physique à venir calculé, d'au moins un du groupe :
- d'instructions de déplacement d'équipement et
- de guidage spatial de personnel ; et
- de fourniture (210) d'au moins une du groupe des instructions de déplacement d'équipement et du guidage spatial de personnel
dans lequel le déplacement physique à venir se rapporte à ce qui se produira dans le scénario actuel en tant que prochaine action concernant le déplacement des dispositifs ou des personnes ;
dans lequel, pour la fourniture des données d'intervention relatives à l'intervention médicale en cours d'exécution, un processeur de données est configuré pour suivre le type et la phase de l'intervention médicale en cours d'exécution ; dans lequel, pour le suivi du type et de la phase de l'intervention médicale en cours d'exécution, le processeur de données est configuré pour fournir au moins une du groupe : analyse d'intervention sur la base de données d'image d'un scénario actuel ; analyse d'intervention sur la base de données acoustiques comprenant au moins des extraits d'une conversation du personnel d'un scénario actuel ; et données se rapportant au patient ; et
dans lequel, pour la fourniture des données spatiales, le processeur de données est configuré pour fournir au moins l'un du groupe suivant : suivi optique d'objets physiques dans un espace prédéterminé de la pièce sur la base de données d'image optique ; et suivi par marqueurs d'au moins un du groupe du personnel et de l'équipement, dans lequel les marqueurs sont fournis en tant qu'au moins un du groupe de marqueurs optiques, de marqueurs électromagnétiques et de marqueurs acoustiques.

12. Produit de programme informatique comprenant des instructions qui, lorsque le programme est exécuté par un ordinateur, amènent l'ordinateur à réaliser le procédé selon la revendication 11.
